(19) **Europäisches Patentamt**

**European Patent Office**

**Office européen des brevets**

(11) **EP 1 074 278 A1**

(12) **EUROPEAN PATENT APPLICATION**
published in accordance with Art. 158(3) EPC

(43) Date of publication:
07.02.2001 Bulletin 2001/06

(51) Int. Cl.$^7$: **A63B 22/06**, A63B 23/04,
A63B 71/06, A61B 5/05

(21) Application number: 99917185.3

(22) Date of filing: 27.04.1999

(86) International application number:
PCT/JP99/02244

(87) International publication number:
WO 99/55427 (04.11.1999 Gazette 1999/44)

(84) Designated Contracting States:
AT BE CH CY DE DK ES FI FR GB GR IE IT LI LU MC NL PT SE

(30) Priority: 28.04.1998 JP 11972898
27.11.1998 JP 33767698

(71) Applicant: Yaman Ltd.
Tokyo 135-0045 (JP)

(72) Inventors:
• YAMAZAKI, Iwao
Tokyo 135-0045 (JP)
• YAMAZAKI, Kimiyo
Tokyo 135-0045 (JP)

(74) Representative:
Newstead, Michael John et al
Page Hargrave
Southgate
Whitefriars
Lewins Mead
Bristol BS1 2NT (GB)

(54) **DEVICE FOR AUTOMATICALLY ADJUSTING LOAD ON AEROBIC EXERCISE MACHINE**

(57) Disclosed is an automatic load control for a load-variable aerobics machine, which permits determination as to how much the machine is to be loaded, how often and how long the so loaded machine is to be used to remove extra amount of bodily fat, and permits the automatic controlling of the machine to follow a given aerobics program while the machine is being used.

It comprises: a bodily impedance measuring means; data input means for inputting individual data pertaining to sex, age, height and weight; means for determining the instantaneous fat rate from the body impedance and the individual data; means for setting a desired fat rate as a target value; means for determining the number of times of exercise and setting the so determined exercise amount for a set exercise amount value; means for comparing the instantaneous fat rate with the set fat rate to determine the amount of exercise required for reducing the difference therebetween; means for determining on the basis of the required amount of excercise and the set exercise amount value an exercise program describing how much heartbeat rate is to be set as a desired one, and how long the aerobics machine is to be used; and means for changing the amount of load on the load-variable aerobics machine to be in conformity with the set heartbeat rate given in the exercise program.

FIG. 5

EP 1 074 278 A1

## Description

### Technical Field

The present invention relates to an aerobics machine which exercise one's arms and legs for the purpose of supplying his body with oxygen and burning his bodily fat such as a treadmill for walking or jogging, a bicycle ergometer for pedaling or a stair climber.

### Background Art

What is aimed at by using an aerobics machine is to strengthen one's heart and lungs for increasing his endurance, and at the same time, remove extra amount of fat, thereby giving him a fatless or muscled nice shape.

For the purpose of controlling for instance, one's weight it is apparently effective that a counselor advises persons taking bodily exercises as to what kind of bodily exercise they should take, and how much and how long they take such bodily exercises.

Such counseling, however, is expensive, and may be inhibitable economically, or may not be available if time not permitting.

In view of the above one object of the present invention is to provide an automatic load control for a load-variable aerobics machine which can take a part of counselor for a person taking a bodily exercise in the hope of removing extra amount of fat, advising him as to how much and how long he takes such a bodily exercise according to a program which can be determined automatically on the basis of individual physical factors, thereby permitting him to continue a bodily exercise in a most efficient way.

### Disclosure of Invention

To attain this object an automatic load control for a load-variable aerobics machine according to one aspect of the present invention comprises: a bodily impedance measuring means having electrodes to be applied to a human body for determining the impedance of the human body; data input means for inputting individual data pertaining to sex, age, height and weight; means for determining the instantaneous fat rate from the body impedance and the individual data; means for determining a desired fat rate and setting the so determined fat rate for a set fat value; means for determining the number of times (or frequency) of exercise and setting the so determined exercise amount for a set exercise amount value; means for comparing the instantaneous fat rate with the set fat rate to determine the amount of exercise required for reducing the difference therebetween; means for determining on the basis of the required amount of exercise and the set exercise amount value an exercise program describing how much the load-variable aerobics machine is to be loaded, and how long the so loaded aerobics machine is to be used; and means for changing the amount of load on the load-variable aerobics machine to be in conformity with the load given in the exercise program, whereby the loading of the aerobics machine may be controlled to consume the amount of exercise required for reaching the set fat value.

An automatic load control for a load-variable aerobics machine according to another aspect of the present invention comprises: a bodily impedance measuring means having electrodes to be applied to a human body for determining the impedance of the human body; data input means for inputting individual data pertaining to sex, age, height and weight; means for determining the instantaneous fat rate from the body impedance and the individual data; means for determining a desired fat rate and setting the so determined fat rate for a set fat value; means for determining the number of times of exercise and setting the so determined exercise amount for a set exercise amount value; means for comparing the instantaneous fat rate with the set fat rate to determine the amount of exercise required for reducing the difference therebetween; means for determining on the basis of the required amount of exercise and the set exercise amount value an exercise program describing how much heartbeat rate is to be set as a desired one, and how long the aerobics machine is to be used; and means for changing the amount of load on the load-variable aerobics machine to be in conformity with the set heartbeat rate given in the exercise program, whereby the loading of the aerobics machine may be controlled to allow the person to consume the amount of exercise required for reaching the set fat value.

### Brief Description of Drawings

Fig.1 shows diagrammatically a treadmill for walking or jogging;
Fig.2 shows diagrammatically a bicycle ergometer for pedaling;
Fig.3 shows diagrammatically a stair climber;

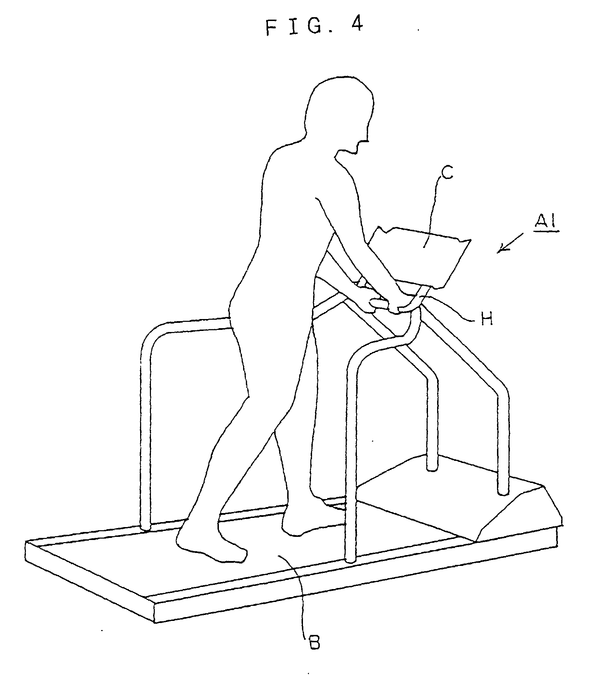

Fig.4 shows diagrammatically the manner in which a person uses a treadmill while measuring the bodily fat rate;

Fig.5 is a block diagram of an automatic load control according to the present invention;

Fig.6 shows a fragment of the grip rod in the automatic load control;

Fig.7 is a block diagram of a bodily impedance measuring circuit in the automatic load control;

Fig.8 is a graphic representation showing how the bodily fat rate decreases to follow the set value therefor; and

Fig.9 is a graphic representation showing how the weight decreases to follow the set value therefor.

**Best Mode of Reducing the Invention into Practice**

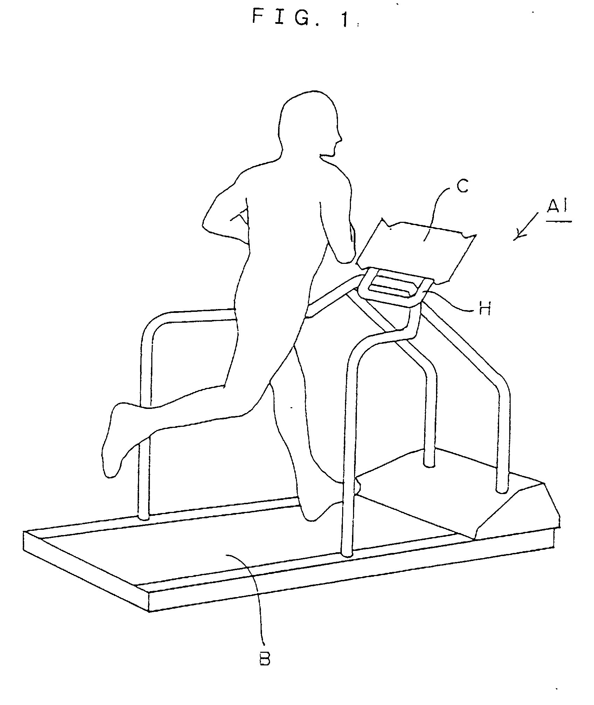

Figs. 1 to 3 show diagrammatically different types of load-variable aerobics machines which can be equipped with an automatic load control according to the present invention.

Referring to Fig.1, a treadmill A1 permits a person to take a jogging or walking exercise by walking or running on its running belt B.

As shown in the drawing, the machine A1 has a color liquid crystal display C mounted on its front side, and it is responsive to finger touch for showing the loading condition, the gradient of the running belt B, the heartbeat rate and other variables.

The loading can be controlled by changing the gradient of the running belt B and the braking torque applied to the belt shafts.

The bodily impedance and the heartbeat rate can be determined while gripping the crossbar fixed to the lower side of the color liquid crystal display C.

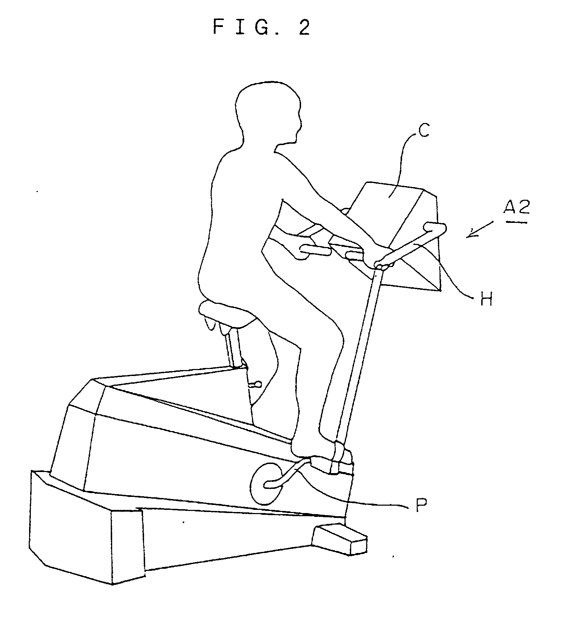

Referring to Fig.2, a bicycle ergometer A2 permits a person to take a pedaling exercise by using its pedals P.

As shown in the drawing, the machine A2 has a color liquid crystal display C mounted on its front side, and it is responsive to finger touch for showing the loading condition, the running speed, the running distance, the heartbeat rate and other variables.

The loading can be controlled by changing the resistance to pedaling, which is caused by the friction of the pedals P, and the braking torque applied to the pedal shafts.

The bodily impedance and the heartbeat rate can be determined while gripping the crossbar fixed to the lower side of the color liquid crystal display C.

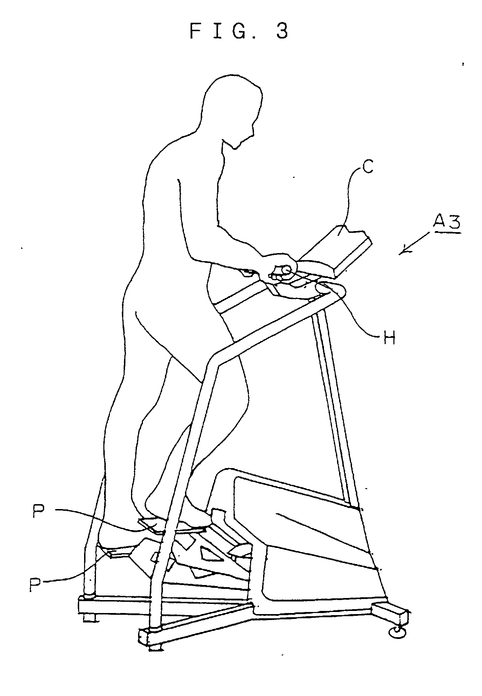

Referring to Fig.3, a stair climber A3 permits a person to take a stair climbing exercise by alternately pushing down its pedals P with his feet.

As shown in the drawing, the machine A3 has a color liquid crystal display C mounted on its front side, and it is responsive to finger touch for showing the loading condition, the climbing speed, the number of steps the person has climbed, the heartbeat rate and other variables.

The loading can be controlled by changing the pedal weight and the braking torque to be applied to the pedal shafts.

The bodily impedance and the heartbeat rate can be determined while gripping the crossbar fixed to the lower side of the color liquid crystal display C.

One example of loading control comprises a shaft having a disk integrally connected thereto and an electromagnet closely adjacent to the disc. As a person is taking a bodily exercise, the shaft rotates, and eddy current is caused by the magnetic flux passing through the disc to cause a braking force to be applied to the disc. The loading can be controlled by controlling the electric current flowing in the electromagnet, thereby controlling the amount of braking effect.

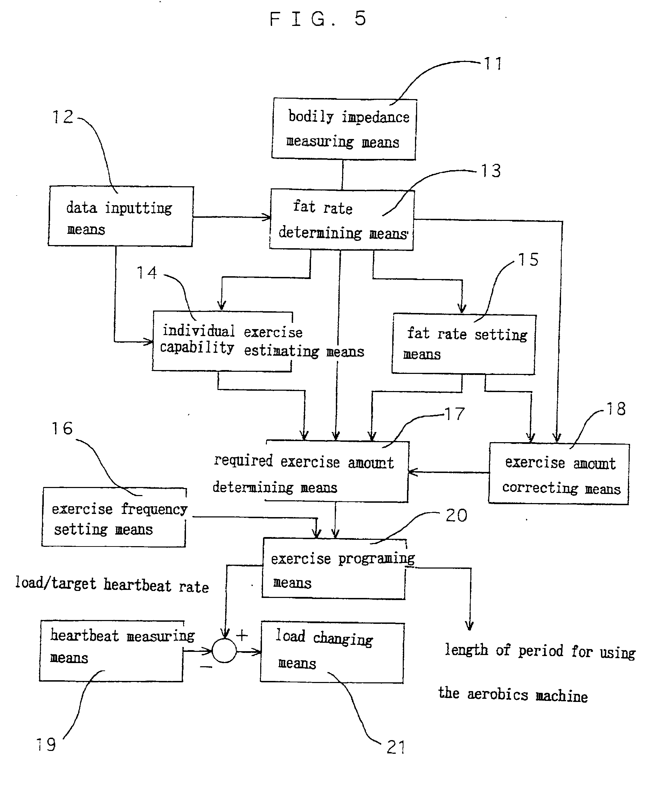

Referring to Fig.5, an automatic load control according to one embodiment of the present invention comprises: a bodily impedance measuring means 11 having electrodes in the form of grip crossbar H to be applied to a human body for determining the impedance of the human body; data input means 12 for inputting individual data pertaining to sex, age, height and weight with the aid of the color liquid crystal display C; means 13 for determining the instantaneous fat rate from the bodily impedance and the individual data; means 14 for estimating one's capability of taking a bodily exercise from the sex, age and the non-fat texture weight (the weight of muscular, bones and other remaining substances determined by subtracting the bodily fat weight from one's weight); means 15 for determining a desired bodily shape and setting the so determined fat rate for a set fat value; means 16 for determining the number of times (or frequency) of exercise and setting the so determined exercise amount for a set exercise amount value; means 17 for comparing the instantaneous fat rate with the set fat rate to determine the amount of exercise (product of load and time) per exercise required for reducing the difference therebetween; means 18 for comparing the instantaneous fat rate with the set fat rate for each time of exercise to modify the amount of exercise; means 19 for determining the number of heartbeats via the electrodes in the form of the grip crossbar H; means 20 for determining on the basis of the required amount of exercise and the set exercise amount value an exercise program describing how much heartbeat rate is to be set as a desired one, and how long the aerobics machine is to be used; and means 21 for comparing the heartbeat rate given by the heartbeat rate determining means 19 with the set value for the desired heartbeat rate, and for changing the amount of load on the load-variable aerobics machine to reduce the difference therebetween in comparison.

While taking a bodily exercise, one's weight can be determined for instance, by using a strain gauge, which may be installed in the aerobics machine.

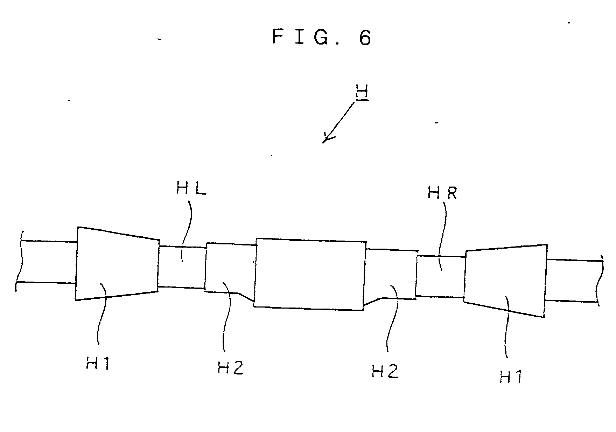

Referring to Fig.6, the grip crossbar H comprises an insulating rod having left and right grips HL and HR integrally connected to its opposite ends, and these grips HL and HR have feeding electrodes H1 and H1 and detecting electrodes H2 and H2 applied therearound. The feeding electrodes H1 and H1 are separated from the detecting electrodes H2 and H2.

These electrodes can be placed at different positions, for examples, the surrounding surface of the color liquid crystal display C.

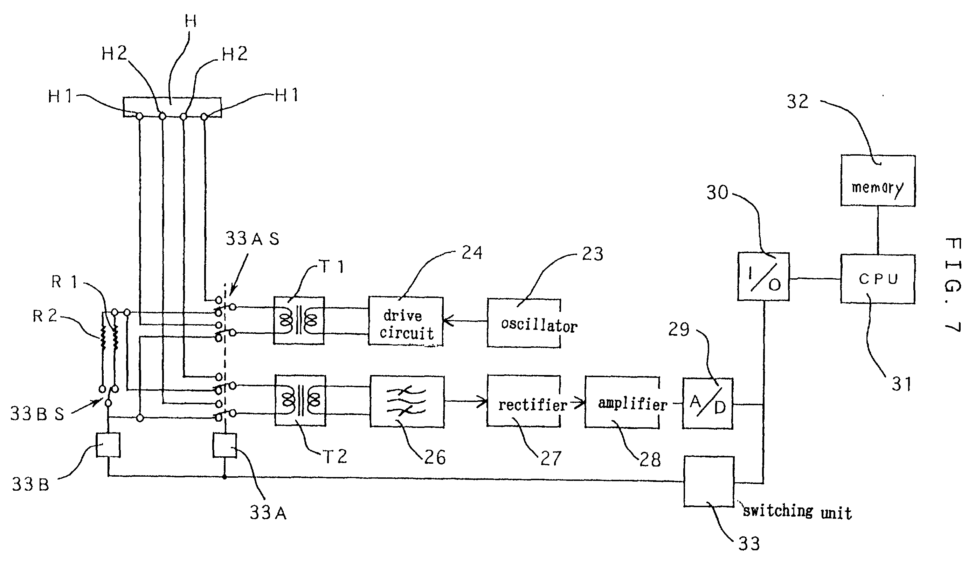

Fig.7 is a block diagram of a measuring circuit of the bodily impedance measuring means 11. As shown in the drawing, an oscillator 23 generates sinusoidal voltage at 50 kHz, which is fed to the feeding electrodes H1 and H1 via a drive circuit 24, a transformer T1 and a switch 33As. When a person holds the left and right grips HL and HR in his hands, an ac voltage appears between the detecting electrodes H2 and H2. The ac voltage is converted to a corresponding dc voltage after passing through the switch 33As, a transformer T2, a band filter 26, a rectifier 27 and an amplifier 28, and then, the dc voltage is directed to a CPU 31 via an A/D converter 29 and an I/O interface 30 after being shaped, level-controlled and offset adjusted. The CPU 31 has a memory 32 associated therewith.

In an attempt to reduce a measurement error which would be caused by the time variability and temperature characteristics of some circuit components the measuring circuit must be calibrated in terms of its input-to-output characteristics prior to measurement of bodily impedance. Specifically the calibration can be effected by using the following equation:

$$Z = k\,V + C0,$$

where Z stands for bodily impedance; V stands for the voltage appearing between the detecting electrodes H2 and H2; k stands for a proportionality factor; and C0 stands for a fixed constant.

The same ac voltage as used in measuring bodily impedance Z is applied across two resistances R1 and R2 of known values to determine the voltages appearing across these known resistances R1 and R2. By substituting the known values of R1 and R2 and the so determined voltages for Z and V in two equations two constants k and C0 can be determined.

To do this the CPU 31 directs a control signal to the switching control 33A via the I/O interface 30 and a switching unit 33 to put the resistances R1 in the measuring circuit. Then, the CPU 31 directs another control signal to the switching control 33B via the I/O interface 30 and the switching unit 33 to put the resistance R2 in the measuring circuit.

The bodily impedance measuring means 11 can determine a bodily impedance according to the following equation 1:

Bodily Impedance = A x Gradient of the Curve x Measured Value + Ordinates

Intersection of the Curve

| A = 0.792 | crossbar | male |
|---|---|---|
| 0.825 | crossbar | female |
| 0.893 | electrode | male |
| 0.95 | electrode | female |

Gradient = (810 - 310)/(Upper Measured Value - Lower Measured Value)

Intersection = 810 - Gradient x Upper Measured Value

The bodily fat rate measuring means 13 can determine a bodily fat rate according to the following equation 2:

Females:

$$\text{Bodily Fat Rate} = (1 - \text{Non-Fat Texture}/\text{Weight}) \times 100$$

$$\text{Non-Fat Texture} = 0.6483 \times (\text{Height} \times \text{Height})/\text{Impedance} + B \times \text{Weight} + 5.091$$

| B = 0.1699 | Height: 150cm or taller | |
|---|---|---|
| 0.12 | Height: below 150cm | Upper Limit of Ideal Weight + 2.5 or more |
| 0.13 | | Upper Limit of Ideal Weight + 2.0 or more |
| 0.14 | | Upper Limit of Ideal Weight + 1.5 or more |
| 0.15 | | Upper Limit of Ideal Weight + 1.0 or more |
| 0.16 | | above Upper Limit of Ideal Weight |
| 0.1699 | | below Upper Limit of Ideal Weight |

$$\text{Upper Limit of Ideal Weight} = (\text{Height} - 100) \times 0.9 \times 1.1$$

Males:

$$\text{Bodily Fat Rate} = (4.95/\text{Bodily Density} - 4.5) \times 100$$

$$\text{Bodily Density} = 1.1554 - (0.0841 \times \text{Weight} \times \text{Impedance})/(\text{Height} \times \text{Height})$$

Means 14 estimates one's capability of taking a bodily exercise, on the basis of which capability the hardness of exercise appropriate for individuals can be determined from the angle of assuring the safety in taking a bodily exercise, still causing a maximum exercise effect.

Usually such one's capability is estimated on the basis of maximum oxygen-intake amount, but in the measurement discussed here it is estimated in terms of non-fat texture (determined by subtracting fat from weight), which is taken as a measure of performance.

It is assumed that the hardness of bodily exercise which can be regarded as a measure of effectively burning the bodily fat is within the range from 60% to 90% of the maximum heartbeat. The maximum heartbeat can be given as follows:

$$\text{Maximum Heartbeat} = 220 - \text{Age}$$

Therefore, the age is one of the factors which are thought of in estimating one's capability of taking a bodily exercise, and the sex is another factor.

Means 15 permits persons to select a desired fat rate in a fat rate menu and set the so selected fat rate as a target value with the aid of the color liquid crystal display P. The desired fat rate for women ranges from 17% to 24% whereas the desired fat rate for men ranges from 14% to 20%.

Means 16 permits persons to determine the number of times of bodily exercise required for attaining the set value for bodily fat by referring to a menu which describes the number of times of bodily exercise in terms of period, for examples, once every day, once a week, three times a week, and how long they must continue the scheduled exercise, for instances, three months, six months or one year. No data of frequency and period are inputted within a given limited time, and then, assumedly appropriate frequency and period are automatically selected and inputted, depending on the difference between the current bodily fat rate and the target value set for the bodily fat rate, so that the number of times of bodily exercise required for attaining the target value is automatically determined and given as a set value.

Means 17 compares the instantaneous bodily fat rate with the set bodily fat rate to determine the amount of bodily exercise (product of load and time) per each bodily exercise, which amount would be required for reducing the difference therebetween. The amount of bodily exercise is given by:

Amount of Bodily Exercise (kcal) = Exercise Hardness (kcal/min.) x Exercise Period (min.)

Amount of bodily Exercise can be increased by increasing exercise hardness and/or exercise period.

In determining the required amount of bodily exercise first, the amount of fat to be consumed is determined from the difference between the current fat rate and the target fat rate set per each bodily exercise. Bodily fat of 1 kg is equivalent to 7000 kcal. The amount of fat to be consumed is expressed in terms of calorie amount, and the amount of exercise is assumed to be equivalent to the so expressed calorie amount. The calorie amount per unit time which can be consumed by taking a bodily exercise can be determined from the basic metabolism and the hardness efficiency at a selected level by following equation 3:

Consumed Calorie Amount per Unit Time = 2.35 x Hardness Efficiency x Basic Metabolic Amount/1440,

where the Basic Metabolic Amount =
Co x Weight x (100 - Bodily Fat Rate)/100 + Cl

| Co = 24.0349 | female at the age of 40 or below |
|---|---|
| 21.951 | female above the age of 40 |
| 27.717 | male at the age of 40 or below |
| 25.333 | male above the age of 40 |
| Cl = 427.64 | female at the age of 40 or below |
| 424.38 | female above the age of 40 |
| 188.21 | male at the age of 40 or below |
| 243.28 | male above the age of 40 |

Hardness efficiency can be given for each level as follows:

| Level 1 | Hardness Efficiency | = 0.825 |
|---------|---------------------|---------|
| Level 2 | | = 0.850 |
| Level 3 | | = 0.875 |
| Level 4 | | = 0.900 |
| Level 5 | | = 0.925 |
| Level 6 | | = 0.950 |
| Level 7 | | = 0.975 |
| Level 8 | | = 1.000 |
| Level 9 | | = 1.025 |
| Level 10 | | = 1.050 |
| Level 11 | | = 1.075 |
| Level 12 | | = 1.100 |
| Level 13 | | = 1.125 |
| Level 14 | | = 1.150 |

Hardness efficiency in bodily exercise is selected from the above by referring to which level is most in conformity with the one's capability of taking a bodily exercise estimated by the means 14.

In case that no level is selected, a standard level is automatically selected.

Once the level has been manually or automatically set, the time required for burning and consuming extra amount of fat can be determined by dividing the amount of bodily exercise expressed in terms of calorie by the calorie per unit time consumed by the bodily exercise.

The length of period thus determined must be 12 minutes long or more; it is a minimum length of period required for starting the burning of bodily fat.

Means 18 compares the instantaneous bodily fat rate with the set bodily fat rate for each exercise to determine the difference therebetween in terms of calorie, thus informing the exercise amount determining means 17 of the difference as representing the exercise amount (expressed in terms of calorie) required for consuming the extra amount of fat. Then, the means 17 makes correction for the exercise amount, if necessary.

As may be understood from the above, persons can determine how much, how often and how long they should take bodily exercises to reach desired bodily fat rate by referring to exercise programs shown on the color liquid crystal display, permitting the variables once set to be corrected while taking bodily exercises. Thus, most effective, adaptive control can be made on bodily exercises.

Means 20 determines on the basis of the required amount of exercise provided by the means 17 and the times of exercise set by the means 16, an exercise program describing how long the aerobics machine is to be used each time of exercise, how much the person is to be loaded in using the machine, and then how much the heartbeat rate is set as target to fulfil the required amount of bodily exercise.

Means 21 compares the instantaneous heartbeat rate given by the means 19 with the one set for the required heartbeat rate, and in order to reduce the difference therebetween means 21 changes the amount of load on the load-variable aerobics machine by changing the gradient of the running belt B, the resistance due to the friction of the pedals P, the braking torque and other variables. Then, the actual heartbeat rate can be retained within the heartbeat zone within which the hardness of exercise which the means 17 determines as appropriate can be met.

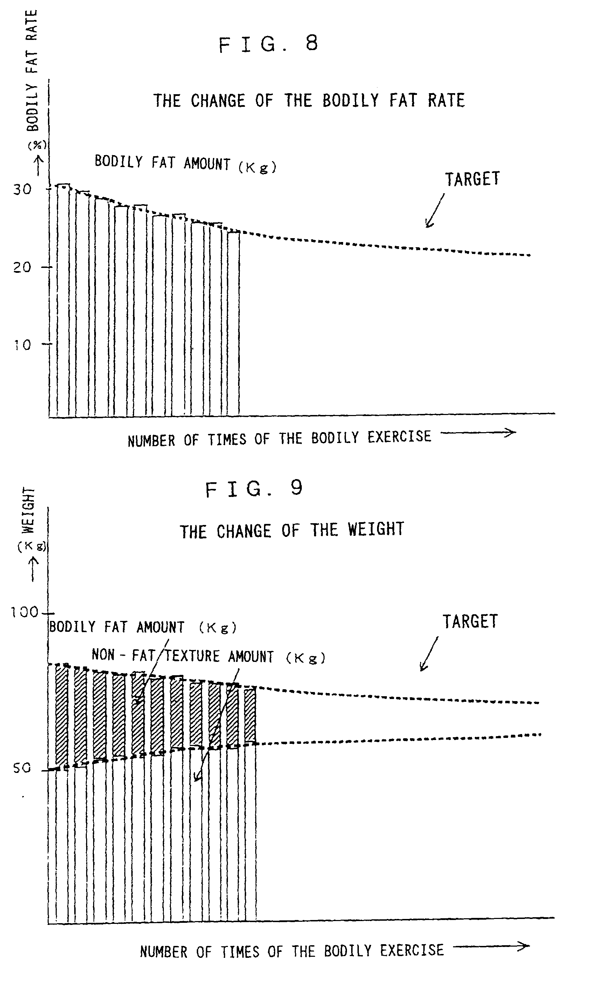

Fig.8 is a graphic representation appearing on the color liquid crystal display C, showing how the bodily fat rate varies relative to the set value. The ordinate of the curve represents bodily fat rate (%) whereas the abscissae of the curve represents the number of times of the bodily exercise. The actual values of bodily fat measured for each time of exercise are plotted along with the set values.

Fig.9 is a graphic representation showing how the weight, the bodily fat amount and the non-fat texture amount vary relative to the corresponding set values. The ordinate of the curves represents weight (kg), which is composed of the bodily fat amount (kg) and the non-fat texture amount (kg) whereas the abscissae of the curve represents the number of times of bodily exercise. The actual values measured for each time of exercise are plotted along with the corresponding set values.

Indirect type of aerobics machines permit the controlling of load in terms of the heartbeat rate as described above. Direct type of aerobics machines, however, attains the controlling of load irrespective of the heartbeat rate.

In the latter case it is necessary that calculations be made to determine how much the machine is loaded, and how long a selected bodily exercise is to be continued for each time of exercise by using the so loaded machine to fulfill the required amount of bodily exercise. Then, an exercise program describing how much the aerobics machine is loaded and how long the aerobics machine is to be used is described. The block diagram of Fig.5 can be applied to both types of aerobics machines.

**Possibility of Industrial Utility**

As is apparent from the above, an automatic load-controlling apparatus for use in an aerobics machine according to the present invention can determine and provide, on the basis of the desired bodily fat rate, an exercise program describing how much the machine is to be loaded, how often and how long the so loaded machine is to be used. Also, the automatic load-controlling apparatus can automatically handle the load controlling means of the aerobics machine so that the machine spontaneously follow the loading condition derived from the exercise program, thus assuring that persons are permitted to take bodily exercise for removing extra amount of bodily fat in a most adaptive, effective way without being disturbed by controlling the machine.

**Claims**

1. An automatic load control for a load-variable aerobics machine comprising:

   a bodily impedance measuring means having electrodes to be applied to a human body for determining the impedance of the human body;
   data input means for inputting individual data pertaining to sex, age, height and weight;
   means for determining the instantaneous fat rate from the body impedance and the individual data;
   means for determining a desired fat rate and setting the so determined fat rate for a set fat value: means for determining the number of times of exercise and setting the so determined exercise amount for a set exercise amount value;
   means for comparing the instantaneous fat rate with the set fat rate to determine the amount of exercise required for reducing the difference therebetween;
   means for determining on the basis of the required amount of exercise and the set exercise amount value an exercise program describing how much the load-variable aerobics machine is to be loaded, and how long the so loaded aerobics machine is to be used; and
   means for changing the amount of load on the load-variable aerobics machine to be in conformity with the load given in the exercise program. whereby the loading of the aerobics machine may be controlled to consume the amount of exercise required for reaching the set fat value.

2. An automatic load control for a load-variable aerobics machine comprising:

   a bodily impedance measuring means having electrodes to be applied to a human body for determining the impedance of the human body;
   data input means for inputting individual data pertaining to sex, age, height and weight;
   means for determining the instantaneous fat rate from the body impedance and the individual data:
   means for determining a desired fat rate and setting the so determined fat rate for a set fat value:
   means for determining the number of times of exercise and setting the so determined exercise amount for a set exercise amount value:
   means for comparing the instantaneous fat rate with the set fat rate to determine the amount of exercise required for reducing the difference therebetween:
   means for determining on the basis of the required amount of exercise and the set exercise amount value an exercise program describing how much heartbeat rate is to be set as a desired one, and how long the aerobics machine is to be used; and
   means for changing the amount of load on the load-variable aerobics machine to be in conformity with the set heartbeat rate given in the exercise program, whereby the loading of the aerobics machine may be controlled to allow the person to consume the amount of exercise required for reaching the set fat value.

F I G. 1.

FIG. 2

F I G. 3

FIG. 4

C

Al

H

B

# F I G. 5

FIG. 6

# FIG. 7

# F I G . 8

## THE CHANGE OF THE BODILY FAT RATE

BODILY FAT RATE (%)

BODILY FAT AMOUNT (Kg)

TARGET

30

20

10

NUMBER OF TIMES OF THE BODILY EXERCISE ⟶

# F I G . 9

## THE CHANGE OF THE WEIGHT

WEIGHT (Kg)

100

BODILY FAT AMOUNT (Kg)

NON - FAT/TEXTURE AMOUNT (Kg)

TARGET

50

NUMBER OF TIMES OF THE BODILY EXERCISE ⟶

# INTERNATIONAL SEARCH REPORT

| International application No. |
| --- |
| PCT/JP99/02244 |

**A. CLASSIFICATION OF SUBJECT MATTER**
Int.Cl⁶ A63B22/06, 23/04, 71/06, A61B5/05

According to International Patent Classification (IPC) or to both national classification and IPC

**B. FIELDS SEARCHED**

Minimum documentation searched (classification system followed by classification symbols)
Int.Cl⁶ A63B22/06, 23/04, 71/06, A61B5/05

Documentation searched other than minimum documentation to the extent that such documents are included in the fields searched

| | | | |
| --- | --- | --- | --- |
| Jitsuyo Shinan Koho | 1922-1996 | Toroku Jitsuyo Shinan Koho | 1994-1999 |
| Kokai Jitsuyo Shinan Koho | 1971-1999 | Jitsuyo Shinan Toroku Koho | 1996-1999 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP, 6-36839, Y2 (YAMAN Ltd.), 28 September, 1994 (28. 09. 94), Full text ; Figs. 1 to 5 (Family: none) | 1, 2 |
| Y | JP, 8-126632, A (Omron Corp.), 21 May, 1996 (21. 05. 96), Full text ; Figs. 1 to 12 (Family: none) | 1, 2 |
| | JP, 8-280840, A (Matsushita Electric Works,Ltd.), 29 October, 1996 (29. 10. 96), | |
| Y | Full text ; Figs. 1 to 9 (Family: none) | 2 |
| A | Full text ; Figs. 1 to 9 (Family: none) | 1 |

☐ Further documents are listed in the continuation of Box C.  ☐ See patent family annex.

| | |
| --- | --- |
| *       Special categories of cited documents: | "T"   later document published after the international filing date or priority |
| "A"   document defining the general state of the art which is not | date and not in conflict with the application but cited to understand |
| considered to be of particular relevance | the principle or theory underlying the invention |
| "E"   earlier document but published on or after the international filing date | "X"   document of particular relevance; the claimed invention cannot be |
| "L"   document which may throw doubts on priority claim(s) or which is | considered novel or cannot be considered to involve an inventive step |
| cited to establish the publication date of another citation or other | when the document is taken alone |
| special reason (as specified) | "Y"   document of particular relevance; the claimed invention cannot be |
| "O"   document referring to an oral disclosure, use, exhibition or other | considered to involve an inventive step when the document is |
| means | combined with one or more other such documents, such combination |
| "P"   document published prior to the international filing date but later than | being obvious to a person skilled in the art |
| the priority date claimed | "&"   document member of the same patent family |

| Date of the actual completion of the international search | Date of mailing of the international search report |
| --- | --- |
| 22 July, 1999 (22. 07. 99) | 3 August, 1999 (03. 08. 99) |

| Name and mailing address of the ISA/ | Authorized officer |
| --- | --- |
| Japanese Patent Office | |
| Facsimile No. | Telephone No. |

Form PCT/ISA/210 (second sheet) (July 1992)